# EUROPEAN PATENT APPLICATION

(11) **EP 3 376 230 A1**
(43) Date of publication of application: **19.09.2018**
(21) Application number: 17382127.3
(22) Date of filing: 13.03.2017
(51) Int. Cl.: G01N 33/68

(54) **IDENTIFICATION OF SIGNATURES FOR NEURODEGENERATION DISEASES DIAGNOSES**

(71) Applicant: Biocross, S.L., 47151 Boecillo-Valladolid (ES)
(72) Inventor: VEIGA HERREROS, Sergio, 47151 Boecillo (Valladolid) (ES); RODRIGUEZ MARTÍN, Andrés, 47151 Boecillo (Valladolid) (ES); GIL DE GÓMEZ SESMA, Luis, 47151 Boecillo (Valladolid) (ES)
(74) Representative: Hoffmann Eitle

(57) **Abstract**

The present invention is directed to diagnostic methods for differentiating Alzheimer's Disease (AD) patients and Frontotemporal Dementia (FTD) patients versus Healthy Controls. The invention also provides methods for distinguishing between these two neurodegenerative diseases AD and FTD, a method for monitoring the progression of AD and/or FTD, a method for determining the efficacy of an AD and/or FTD therapy and a method for screening compounds to be used against AD and/or FTD.

## Description

### FIELD OF THE INVENTION

The present invention relates to the field of diagnostics and, more in particular the non-invasive diagnosis of Alzheimer's Disease (AD) and Frontotemporal Dementia (FTD) patients showing different plasma metabolomic profiles. This invention further relates to the use of plasma metabolomics biomarkers to differentiate AD or FTD from Healthy Control (HC) or AD from FTD with different circulating metabolite signatures.

### BACKGROUND OF THE INVENTION

Alzheimer's disease (AD) is a progressive degenerative disease of the central nervous system characterized by progressive and increasing memory loss, followed by loss of control of limbs and bodily functions and eventual death. It is by far the most common cause of dementia affecting 1 to 6% of people over the age of 65 years and between 10 to 20% of those over 80. AD is distinguished from other types of dementia by several pathological features, including the progressive appearance in the brain of the patients of senile plaques in the extracellular space between neurons. The plaques have central cores of amyloid deposits formed mainly by fibrils of a 40-42 amino acids peptide referred to amyloid β peptide (Aβ) surrounded by degenerated neuritis and glial cells. This peptide results from the proteolytic processing of a precursor protein called β amyloid precursor protein (βAPP). AD can be classified according to the age of appearance as early onset (age under 60 years) and late onset (age above 60 years), according to the existence of an autosomic dominant inheritance, as familiar AD or sporadic AD respectively. Early onset familiar forms of AD can be associated to known mutation in the genes coding for βAPP, presenilin 1 and presenilin 2 (located, respectively, on chromosomes 21, 14 and 1).

These classifications are not mutually exclusive. The most frequent forms are sporadic lateonset forms. In clinical praxis, diagnosis of AD is carried out using clinical criteria based on the presence of typical clinical hallmarks and the exclusion of other types of dementia using neuroimaging techniques and blood analysis. Using these criteria, diagnostic reliability is acceptable although, according to studies done using brain autopsy, between 10-20% of the patients diagnosed with AD suffered from a different disease.

Frontotemporal dementia (FTD) is another neurodegenerative disease. It is the second most prevalent dementia of patients below age 65. The disease is often misdiagnosed in the early stage, either as a psychiatric disorder, or as a different type of dementia such as Alzheimer's disease (AD).

The underlying pathological spectrum of FTD, frontotemporal lobar degeneration (FTLD), can be divided in 2 main subtypes characterized by either tau or TDP-43 accumulations.

According with the Alzheimer's Association, the disorders grouped under FTD fall into three subtypes:
- Behavior variant frontotemporal dementia (bvFTD): This condition is characterized by prominent changes in personality, interpersonal relationships and conduct that often occur in people in their 50s and 60s, but can develop as early as their 20s or as late as their 80s. In bvFTD, the nerve cell loss is most prominent in areas that control conduct, judgment, empathy and foresight, among other abilities.
- Primary progressive aphasia (PPA): This is the second major form of frontotemporal degeneration that affects language skills, speaking, writing and comprehension. PPA normally comes on in midlife, before age 65, but can occur in late life also. The two most distinctive forms of PPA have somewhat different symptoms.
- Disturbances of motor (movement or muscle) function: There are three disorders that are a part of the frontotemporal degeneration spectrum that produce changes in muscle or motor functions with or without behavior (bvFTD) or language (PPA) problems; Amyotrophic lateral sclerosis (ALS), Corticobasal syndrome, and Progressive supranuclear palsy (PSP).

Methods are known in the prior art to diagnose AD by detecting the levels of biomarkers present in the brain or cerebrospinal fluid (CSF) of patients. Different biomarkers have been characterized whose determination is carried out in CSF. CSF reflects directly the composition of the extracellular space of the central nervous system and thus, provides higher concentrations of biomarkers. The cerebrospinal fluid (CSF) biomarkers for AD, i.e. (p) Tau and amyloid beta-42, appear to be of limited value for the diagnosis of clinical FTD. While structural MR scans can be used to detect frontal lobe or temporal lobe atrophy, the early stages of FTD are normally not detectable.

However, CSF can only be retrieved by means of lumbar puncture, which is not a routine diagnostic method easily accepted by patients suffering from dementia, let alone in patients with memory disorders. Thus, there is a need for AD biomarkers which can be detected in samples which can be non-invasively retrieved from the body. Suitable AD biomarkers described in the prior art and which can be detected in plasma include (i) markers derived from the amyloid plaque, (ii) autoantibodies against Aβ o βAPP, (iii) inflammatory markers such IL-6, its receptor or gp130, C-reactive protein or oxidative stress (isoprostanes), (iv) markers of lipidic metabolism (apoE, oxysterols) and (v) vascular disease markers (homocysteine, lipoprotein b C1q) (Scheuner D. et al., Nature Med, 1996, 2, 864-870). WO2011/143574, WO2013/153461 and WO2015/006489 are also describing different biomarkers found in non-invasive samples.

Regarding FTD, WO2015/152724 describes a method of classifying the FTD status of an individual, comprising determining the concentration of a panel of biomarkers selected from a biological sample from said individual. However, among all the biomarkers that they found relevant only one seems to be correlated in CSF and serum samples.

US2010/0136573 provides methods to determine if a mammal has a neurodegenerative disease based on TDP-43 levels and/or TDP-43 cleavage products in a biological fluid. However, the method although useful in non-invasive methods (plasma and serum) is not able to classify which neurodegenerative disease the patient is suffering from.

US2012/0015381 is describing a differential method for diagnosis AD or FTD based in serum biomarkers with statistical values but the number of samples used in this study for each group is very limited.

However, none of these non-invasive biomarkers panels described so far have reached the right level of precision to enable a reliable diagnosis, and even less reliable if it is in a preclinical stage. Consequently, so far no evidence has been incorporated into clinical practice.

We present here a multiparametric biomarker panel that fulfills a long-felt need in the art with strong statistic potency based in a high number of samples, useful for diagnosing and predicting the onset of both Alzheimer's disease and FTD based on the analysis of bodily fluids, which can be easily obtainable by non-invasive means.

### BRIEF DESCRIPTION OF THE INVENTION

The authors of the present invention have identified a series of metabolic markers present in the plasma samples collected from subjects previously diagnosed with Alzheimer Disease (AD) or Frontotemporal dementia (FTD, (variants bvFTD and PPA). These metabolic markers selected are significantly differentiated between Healthy Controls (HC) and AD or FTD and between AD and FTD. These metabolic markers can then be used in a non-invasive diagnostic method identifying and classifying patients with AD or FTD patients versus HC.

In a first aspect, the invention relates to a diagnostic method to distinguish between AD patients or FTD patients versus non-affected patients based in three different plasma biomarkers profiles. The diagnostic method for detecting AD patients versus HC is by determining in a biological sample of a subject the levels of at least one, preferably all, of the 10 biomarkers described in table 1. The diagnostic method for detecting FTD patients versus HC patients is by determining in a biological sample of a subject the levels of at least one, preferably all, of the different 10 biomarkers described in table 2. The diagnostic method for distinguishing AD patients versus FTD patients is by determining in a biological sample of a subject the levels of at least one, preferably all, of the 4 biomarkers described in table 3.

The diagnostic methods of AD versus HC, FTD versus HC and AD versus FTD is by determining in a biological sample of a subject suspected of suffering from AD or FTD, the levels of at least one metabolic marker or markers described in table 1, table 2 and table 3 wherein
- increased level(s) of Ornithine, Putrescine, PC aa C34:3 and/or PC ae C30:0 from table 1 in AD with respect to HC or with respect a reference value are indicative that the subject suffers from AD; increased level(s) of L-Alanine, Ornithine, C18:2 and/or PC ae C30:0 from table 2 in FTD with respect to HC or with respect a reference value are indicative that the subject suffers from FTD; and increased level(s) of PC ae C34:3)from table 3 in AD with respect to FTD or with respect a reference value are indicative that the subject suffers from AD; and /or
- decreased level(s) of L-Arginine, L-Glutamic Acid, Alpha-AAA, LysoPC a C18:0 and/or PC ae C36:0 from table 1 in AD with respect to HC or with respect a reference value are indicative that the subject suffers from AD; decreased level(s) of L-Arginine, L-Lysine, L-Tryptophan, PC aa C34:2, PC aa C42:2 and/or PC ae C38:0 from table 2 in FTD with respect to HC or with respect a reference value are indicative that the subject suffers from FTD; and decreased level(s) of L-Glutamic Acid and/or C18:2 from table 3 in AD with respect to FTD or with respect a reference value are indicative that the subject suffers from AD.

In a second aspect, the invention relates to a method for determining the efficacy of a therapy for AD or FTD comprising determining in a biological sample of a subject suffering from AD or FTD and having been treated with said therapy the levels of at least one metabolic marker or markers described in table 1, table 2 wherein
- decreased level(s) of Ornithine, Putrescine, PC aa C34:3 and/or PC ae C30:0 from table 1 in AD with respect to HC or with respect a reference value are indicative that said therapy is effective against AD; decreased level(s) of L-Alanine, Ornithine, C18:2 and/or PC ae C30:0 from table 2 in FTD with respect to HC or with respect a reference value are indicative that said therapy is effective against FTD; and /or
- increased level(s) of L-Arginine, L-Glutamic Acid, Alpha-AAA, LysoPC a C18:0 and/or PC ae C36:0 from table 1 in AD with respect to HC or with respect a reference value are indicative that said therapy is effective against AD; increased level(s) of L-Arginine, L-Lysine, L-Tryptophan, PC aa C34:2, PC aa C42:2 and/or PC ae C38:0 from table 2 in FTD with respect to HC or with respect a reference value are indicative that said therapy is effective against FTD.

In a third aspect, the invention relates to a method for the identification of compounds for AD or FTD therapy comprising determining in a biological sample of a subject having been treated with a test compound the levels of at least one metabolic marker or markers described in table 1, table 2 wherein
- decreased level(s) of Ornithine, Putrescine, PC aa C34:3 and/or PC ae C30:0 from table 1 in AD with respect to HC or with respect a reference value are indicative that the compound is effective for the treatment of AD; decreased level(s) of L-Alanine, Ornithine, C18:2 and/or PC ae C30:0 from table 2 in FTD with respect to HC or with respect a reference value are indicative that the compound is effective for the treatment of FTD; and /or
- increased level(s) of L-Arginine, L-Glutamic Acid, Alpha-AAA, LysoPC a C18:0 and/or PC ae C36:0 from table 1 in AD with respect to HC or with respect a reference value are indicative that the compound is effective for the treatment of AD,; increased level(s) of L-Arginine, L-Lysine, L-Tryptophan, PC aa C34:2, PC aa C42:2 and/or PC ae C38:0 from table 2 in FTD with respect to HC or with respect a reference value are indicative that the compound is effective for the treatment of FTD.

In a fourth aspect, the invention relates to a method for monitoring the progression of AD or FTD diseases comprising determining in a biological sample of a subject, without having been treated or having been treated with a therapy indicated for AD or FTD, the levels of at least one metabolic marker or markers described in table 1 and table 2 wherein
- increased level(s) of Ornithine, Putrescine, PC aa C34:3 and/or PC ae C30:0 from table 1 with respect to a reference value determined in a sample from the same subject at an earlier time point are indicative that AD condition is progressing; increased level(s) of L-Alanine, Ornithine, C18:2 and/or PC ae C30:0 from table 2 with respect to a reference value determined in a sample from the same subject at an earlier time point are indicative that FTD condition is progressing; and/or
- decreased level(s) of L-Arginine, L-Glutamic Acid, Alpha-AAA, Lyso PC a C18:0 and/or PC ae C36:0 from table 1 with respect to a reference value determined in a sample from the same subject at an earlier time point are indicative that AD condition is progressing; decreased level(s) of L-Arginine, L-Lysine, L-Tryptophan, PC aa C34:2, PC aa C42:2 and/or PC ae C38:0 from table 2 with respect to a reference value determined in a sample from the same subject at an earlier time point are indicative that FTD condition is progressing.

It is noted that all of the above methods can be complemented by detecting the presence or absence of APOE4 in the biological sample.

### BRIEF DESCRIPTION OF DRAWINGS

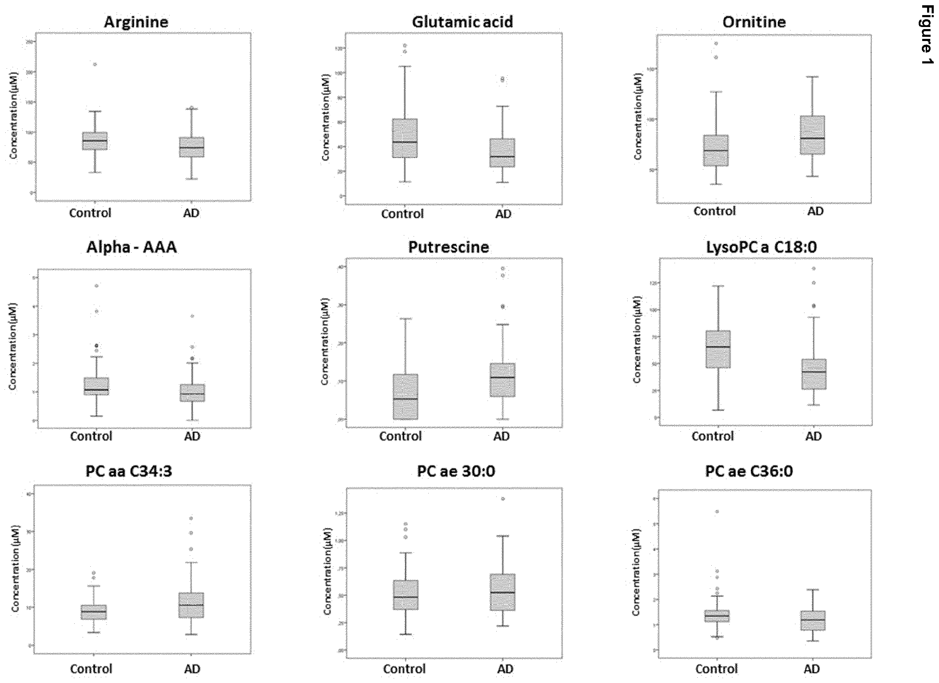

FIG. 1 Box plots show the concentration (µm) of each of the 9 metabolites included in the final algorithm to discriminate healthy controls (Control) from patients diagnosed with Alzheimer's Disease (AD). Horizontal lines within each box represent the median of the sample, while the bottom and top of each box represent the lower and upper quartiles respectively. Whiskers represent the lower and highest value of each set of data, excluding outliers. Outliers are represented as small circles.

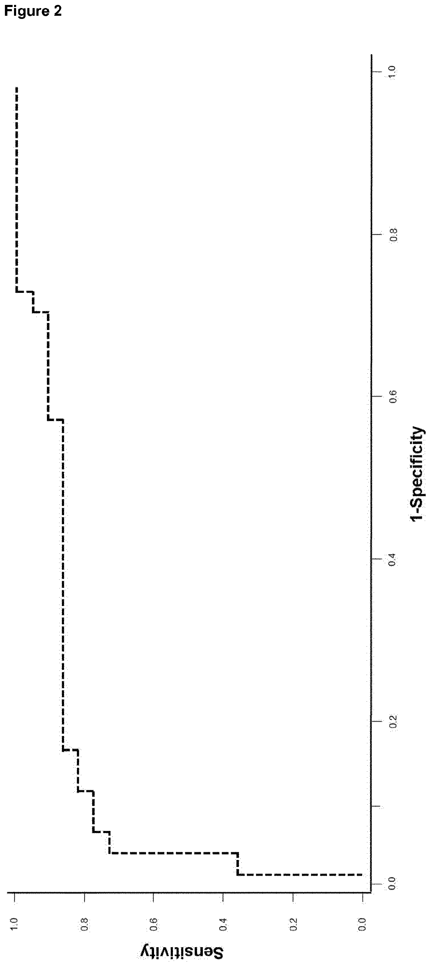

FIG. 2 Receiver Operator Curve (ROC) is shown for the discrimination of AD vs HC, showing sensitivity (i.e., true positive rate) versus 1-specificity (i.e., false positive rate). The dots line represents the Area under the Curve (AUC, 0,96).

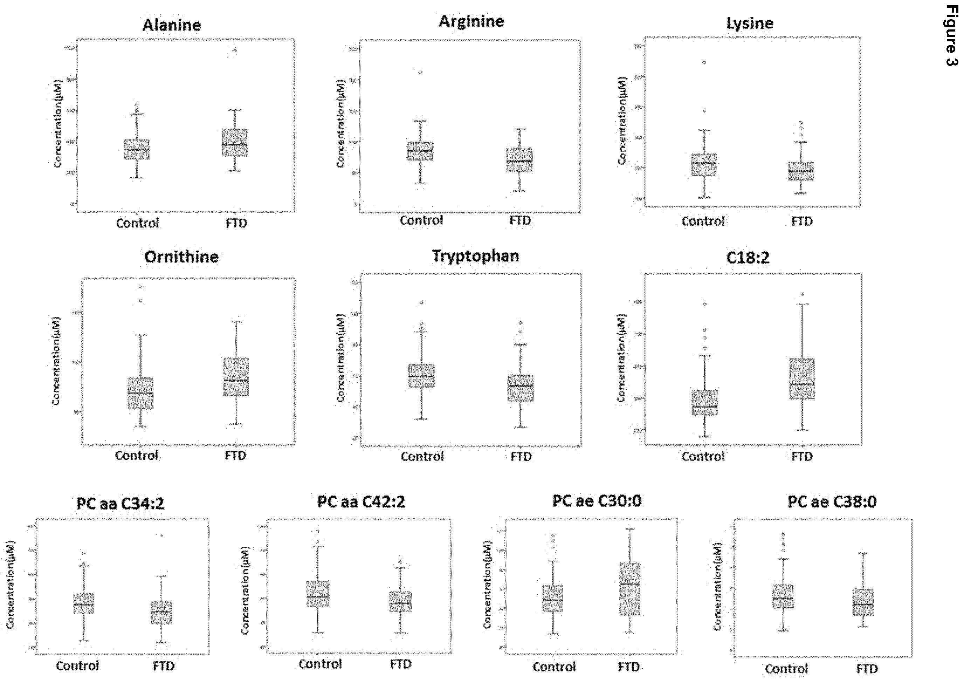

FIG. 3 Box plots show the concentration (µm) of each of the 10 metabolites included in the final algorithm to discriminate healthy controls (Control) from patients diagnosed with Frontotemporal Dementia (FTD). Horizontal lines within each box represent the median of the sample, while the bottom and top of each box represent the lower and upper quartiles respectively. Whiskers represent the lower and highest value of each set of data, excluding outliers. Outliers are represented as small circles.

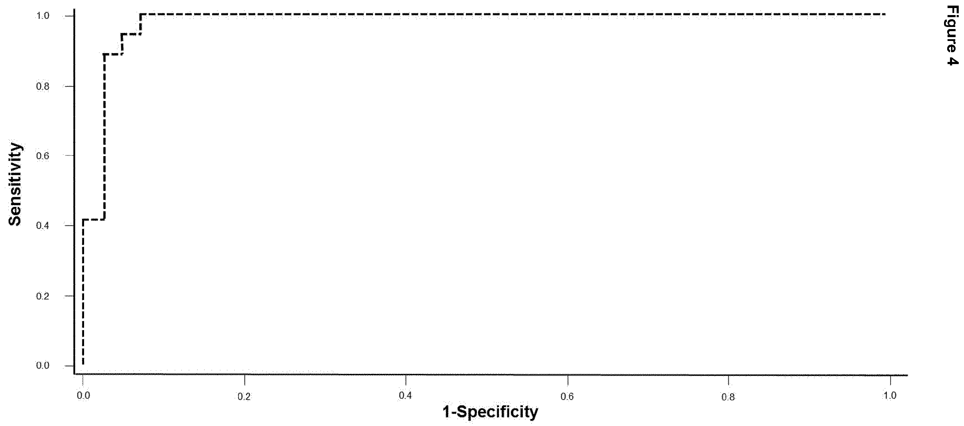

FIG. 4 Receiver Operator Curve (ROC) is shown for discrimination of FTD vs AD, showing sensitivity (i.e., true positive rate) versus 1-specificity (i.e., false positive rate). The dots line represents the Area under the Curve (AUC, 0, 92).

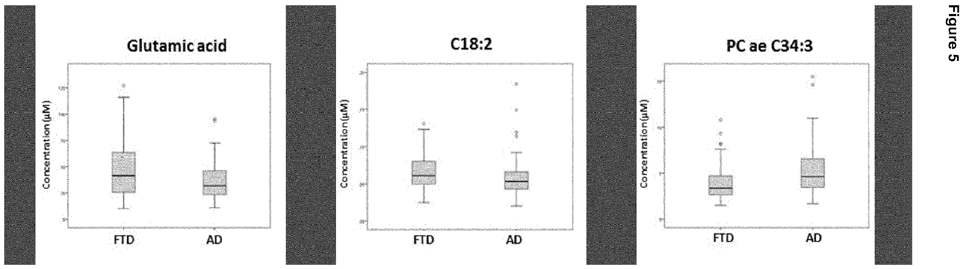

FIG. 5 Box plots show the concentration (µm) of each of the 3 metabolites included in the final algorithm to discriminate patients diagnosed with Frontotemporal dementia (FTD) from patients diagnosed with Alzheimer's Disease (AD). Horizontal lines within each box represent the median of the sample, while the bottom and top of each box represent the lower and upper quartiles respectively. Whiskers represent the lower and highest value of each set of data, excluding outliers. Outliers are represented as small circles.

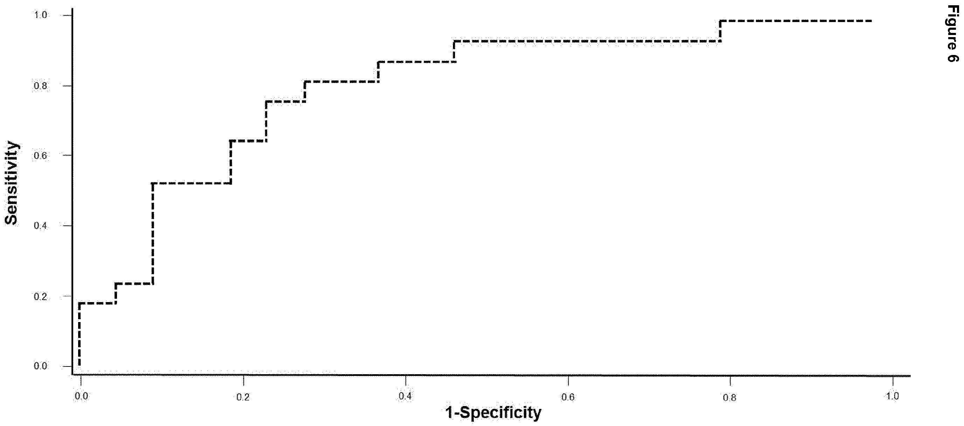

FIG. 6 Receiver Operator Curve (ROC) is shown curve for the discrimination of FTD vs AD, showing sensitivity (i.e., true positive rate) versus 1-specificity (i.e., false positive rate). The dots line represents the Area under the Curve (AUC, 0, 79).

### DESCRIPTION OF THE INVENTION

### Diagnostic methods of the Invention

Before the present methods are described, it is to be understood that this invention is not limited to particular methods, and experimental conditions described, methods and conditions may vary. It is also to be understood that the terminology used herein is for purposes of describing particular embodiments only, and is not intended to be limiting, since the scope of the present invention will be limited only in the appended claims.

In a first aspect the invention relates to a diagnostic method to distinguish AD or FTD patient subjects and to distinguished between AD and FTD patient subjects, hereinafter "first diagnostic method of the invention" comprising determining in a biological sample of said subject the levels of at least one metabolic marker described in table 1 or table 2 or table 3 and comparing the levels of said marker or markers with respect the levels of the same marker or markers in a HC patient or with respect to the same marker or markers in an FTD or in an AD patient or with respect to a reference value as described through-out the present specification, wherein the subject is classified as suffering AD or FTD respectively as explained throughout the specification and in the examples.

The term "diagnosis", as used herein, refers both to the process of attempting to determine and/or identify a possible disease in a subject, i.e. the diagnostic procedure, and to the opinion reached by this process, i.e. the diagnostic opinion. As such, it can also be regarded as an attempt at classification of an individual's condition into separate and distinct categories (such as predicting the "increasing risk" of suffering a disease, meaning "increasing risk" as an increased chance of developing or acquiring a disease compared with a normal individual) that allow medical decisions about treatment and prognosis to be made. It is to be understood that the method, in a preferred embodiment, is a method carried out *in vitro,* i.e. not practiced on the human or animal body. In particular, the diagnosis to determine AD or FTD patients, relates to the capacity to identify and classify AD or FTD patients. This diagnosis, as it is understood by a person skilled in the art does not claim to be correct in 100% of the analyzed samples.

However, it requires that a statistically significant amount of the analyzed samples are classified correctly. The amount that is statistically significant can be established by a person skilled in the art by means of using different statistical tools; illustrative, non-limiting examples of said statistical tools include determining confidence intervals, determining the p-value, the Chi-Square test discriminating functions, etc. Preferred confidence intervals are at least 90%, at least 97%, at least 98%, at least 99%. The p-values are, preferably less than 0.1, less than 0.05, less than 0.01, less than 0.005 or less than 0.0001. The teachings of the present invention preferably allow correctly diagnosing in at least 60%, in at least 70%, in at least 80%, or in at least 90% of the subjects of a determining group or population analyzed.

The term "Alzheimer's disease" or "AD", as used herein, refers a mental deterioration associated with specific degenerative brain disease that is characterized by senile plaques, neuritic tangles and progressive neuronal loss which manifests clinically in progressive memory deficits, confusion, behavioral problems, inability to care for oneself, gradual physical deterioration and, ultimately, death. As used herein, this term is intended to include all the stages (such as preclinical stage) of the disease.

The term "Frontotemporal dementia" or "FTD", as used herein refers to a disorder caused by the degeneration of the brain's frontal lobes. Diagnostic criteria according to the Lund-Manchester criteria include insidious onset and gradual progression, early decline in social interpersonal conduct, early impairment in regulation of personal conduct, early emotional blunting, and early loss of insight. Symptoms of FTD generally appear between the ages of 45 and 65 years of age. As used herein, this term is intended to include all the stages (such as preclinical stage) and subtypes of the disease.

The terms "subject", "patient" or "individual"' are used herein interchangeably to refer to all the animals classified as mammals and includes but is not limited to domestic and farm animals, primates and humans, for example, human beings, non-human primates, cows, horses, pigs, sheep, goats, dogs, cats, or rodents. Preferably, the subject is a male or female human being of any age or race.

The term "metabolic marker" or "metabolite" or "biomarker", are used herein interchangeably to refers to small molecule compounds, such as substrates for enzymes of metabolic pathways, intermediates of such pathways or the products obtained by a metabolic pathway, the occurrence or amount of which is characteristic for a specific situation, for example AD or FTD. The metabolic markers useful for the first diagnostic method of the invention are those defined in table 1, table 2 and table 3. Table1, table 2 and table 3 contain the abbreviated common names of the metabolites and the lipid family. The lipid family is further described by the reference number of said lipid family in the LIPID MAPS structure database (http://www.lipidmaps.org/data/databases.html) using the LIPID MAPS Classification System (Fahy E. et al., Journal of Lipid Research 2009, 50: S9-S14). The lipid metabolic markers of table 1, table 2 and table are intended to refer to any isomer thereof, including structural and geometric isomers. The term "structural isomer", as used herein, refers to any of two or more chemical compounds, having the same molecular formula but different structural formulas. The term "geometric isomer" or "stereoisomer" as used herein refers to two or more compounds which contain the same number and types of atoms, and bonds (i.e., the connectivity between atoms is the same), but which have different spatial arrangements of the atoms, for example cis and trans isomers of a double bond, enantiomers, and diastereomers. The abbreviated common name of the amino acid or protein corresponds to the Amino Acid name or Protein to which it belongs followed by an accession number described in the Human Metabolome Database HMDB (http://www.hmdb.ca).

**Table 1: Plasma metabolites significantly differentiated between Healthy Controls and Alzheimer's patients.**

| **Abbreviated or Common name** | **IUPAC name (or Traditional name) and HMDB accession number or Subclass or UniProt accession number** |
|---|---|
| L-Arginine | (2S)-2-amino-5-carbamimidamidopentanoic acid (HMDB00517) |
| L-Glutamic Acid | (2S)-2-aminopentanedioic acid (HMDB00148) |
| Ornithine | (2S)-2,5-diaminopentanoic acid (HMDB00214) |
| Aminoadipic acid (AAA) | 2-aminohexanedioic acid(HMDB00510) |
| Putrescine | butane-1,4-diamine (HMDB01414) |
| Lyso PC a C18:0 | 1-octadecanoyllysolecithin (HMDB10384) |
| PC aa C34:3 | Diacylglycerophosphocholines [GP0101] |
| PC ae C30:0 | Diacylglycerophosphocholines [GP0101] |
| PC ae C36:0 | Diacylglycerophosphocholines [GP0101] |
| APOE4 | Apolipoprotein-E (P02649) isoform E4 |

In a particular embodiment, the first diagnostic method of the invention comprises determining the levels of L-Arginine. It is noted that a decrease of said marker with respect to HC or with respect to a reference value is indicative that the subject suffers from AD.

In a particular embodiment, the first diagnostic method of the invention comprises determining the levels of L-Glutamic Acid. It is noted that a decrease of said marker with respect to HC or with respect to a reference value is indicative that the subject suffers from AD.

In a particular embodiment, the first diagnostic method of the invention comprises determining the levels of Ornithine. It is noted that an increase of said marker with respect to HC or with respect to a reference value is indicative that the subject suffers from AD.

In a particular embodiment, the first diagnostic method of the invention comprises determining the levels of Putrescine. It is noted that an increase of said marker with respect to HC or with respect to a reference value is indicative that the subject suffers from AD.

In a particular embodiment, the first diagnostic method of the invention comprises determining the levels of Lyso PC a C18:0. It is noted that a decrease of said marker with respect to HC or with respect to a reference value is indicative that the subject suffers from AD.

In a particular embodiment, the first diagnostic method of the invention comprises determining the levels of PC aa C34:3. It is noted that an increase of said marker with respect to HC or with respect to a reference value is indicative that the subject suffers from AD.

In a particular embodiment, the first diagnostic method of the invention comprises determining the levels of PC ae C30:0. It is noted that an increase of said marker with respect to HC or with respect to a reference value is indicative that the subject suffers from AD.

In a particular embodiment, the first diagnostic method of the invention comprises determining the levels of PC ae C36:0. It is noted that a decrease of said marker with respect to HC or with respect to a reference value is indicative that the subject suffers from AD.

In a particular embodiment, the first diagnostic method of the invention comprises determining the presence of allele APOE4. It is noted that the presence of said allele is indicative that the subject suffers from AD.

In a preferred embodiment the method further comprises confirming the diagnosis of AD by means of the clinical examination of the patient, preferably by using anamnesis and neuropsychological tests (such as the MiniMental State Examination (MMSE) or clinical dementia Rating (CDR). Blood tests are used to rule out dementia due to other causes such as syphilis, Vitamin B12 deficiency, abnormal thyroid levels or HIV.

It is further noted that Diagnostic accuracy can be increased through the analysis of biomarkers ((p) Tau and amyloid beta-42) in CSF by ELISA; through the analysis of Amyloid beta, Tau or p-Tau in cerebral tissue (by Positron Emision Tomography (PET) using specific radioligands; through the analysis of brain hypometabolism in specific areas of the brain by means of fluoro-D-glucose PET (FdG PET); through the analysis of cerebral blood flow in specific brain areas using single photon emission computed tomography (SPECT), and through the analysis of the atrophy of temporal areas of the brain using magnetic resonance imaging (MRI). None of the above-mentioned diagnostic methods provide a definitive AD diagnosis, which can only be achieved by a brain autopsy.

Currently, there are not disease-modifying treatment for Alzheimer's Disease. Patients diagnosed with AD are treated with drugs aimed to reduce symptomatology. The two most common drugs prescribed for AD patients are acethylcholinesterase inhibitors such as donezepil for mild to moderate AD and NMDA glutamate receptor blocking agents such as mematine, for moderate to severe AD.

The most tested strategies in phase III clinical trials are therapies directed to reduce either the amount or the formation of Aβ. They include the use of antibodies directed to soluble Aβ and/or Aβ plaques in the brain, and inhibitors of beta-secretase (BACE, the enzyme that cleavages the amyloid precursor protein and favors the accumulation of Aβ). All phase III clinical trials with these compounds were directed to mild to moderate stages of the disease and all failed to improve performance in neuropsychological tests. These compounds are now being tested on earlier stages of the disease including asymptomatic phases, with the hope that they will be more effective in stages where the neurodegeneration has not already happened.

Other therapeutic strategies being tested in phase III clinical trials include but are not limited to dietary interventions (for example ketogenic diets or DHA supplements), drugs targeting Tau, or agonist/antagonists of neurotransmitters such as serotonine and dopamine.

**Table 2: Plasma metabolites significantly differentiated between Healthy Controls and Frontotemporal Dementia patients.**

| **Abbreviated or Common name** | **IUPAC name (or Traditional name) and HMDB accession number or Subclass or UniProt accession number** |
|---|---|
| L-Alanine | (2S)-2-aminopropanoic acid (HMDB00161) |
| L-Arginine | (2S)-2-amino-5-carbamimidamidopentanoic acid (HMDB00517) |
| L-Lysine | (2S)-2,6-diaminohexanoic acid (HMDB00182) |
| Ornithine | (2S)-2,5-diaminopentanoic acid (HMDB00214) |
| L-Tryptophan | 2S)-2-amino-3-(1H-indol-3-yl)propanoic acid (HMDB009299) |
| C18:2 | Unsaturated fatty acids [FA0103] |
| PC aa C34:2 | Diacylglycerophosphocholines [GP0101] |
| PC aa (42:2) | Diacylglycerophosphocholines [GP0101] |
| PC ae C30:0 | Diacylglycerophosphocholines [GP0101 |
| PC ae C38:0 | Diacylglycerophosphocholines [GP0101] |

In a particular embodiment, the first diagnostic method of the invention comprises determining the levels of L-Alanine. It is noted that an increase of said marker with respect to HC or with respect to a reference value is indicative that the subject suffers from FTD.

In a particular embodiment, the first diagnostic method of the invention comprises determining the levels of L-Lysine. It is noted that a decrease of said marker with respect to HC or with respect to a reference value is indicative that the subject suffers from FTD.

In a particular embodiment, the first diagnostic method of the invention comprises determining the levels of L-Tryptophan. It is noted that a decrease of said marker with respect to HC or with respect to a reference value is indicative that the subject suffers from FTD.

In a particular embodiment, the first diagnostic method of the invention comprises determining the levels of C18:2. It is noted that an increase of said marker with respect to HC or with respect to a reference value is indicative that the subject suffers from FTD.

In a particular embodiment, the first diagnostic method of the invention comprises determining the levels of PC aa C34:2. It is noted that a decrease of said marker with respect to HC or with respect to a reference value is indicative that the subject suffers from FTD.

In a particular embodiment, the first diagnostic method of the invention comprises determining the levels of PC aa C42:2. It is noted that a decrease of said marker with respect to HC or with respect to a reference value is indicative that the subject suffers from FTD.

In a particular embodiment, the first diagnostic method of the invention comprises determining the levels of PC ae C38:0. It is noted that a decrease of said marker with respect to HC or with respect to a reference value is indicative that the subject suffers from FTD.

In a preferred embodiment the first diagnostic method further comprises confirming the diagnosis of FTD by means of the clinical examination of the patient, preferably using neuropsychological tests (such as the MiniMental State Examination (MMSE) or clinical dementia Rating (CDR)) and anamnesis. Additionally, blood tests are used to rule out dementia due to other causes such as syphilis, Vitamin B12 deficiency, endocrine abnormalities or HIV. CSF biomarker analysis of Amyloid beta 42, Tau and p-Tau can be performed to rule out AD.

Additionally, frontal ad temporal lobes atrophy evaluation by MRI is frequently used to help with FTD diagnosis. Currently, there are not disease-modifying treatments for Frontotemporal dementia. Patients diagnosed with FTD might be treated with drugs aimed to reduce symptomatology. Selective Serotonine Reuptake Inhibitors (SSRI) and Selective Nor-Adrenaline Reuptake inhibitors (SSNRI) antidepresants might be used to treat behavioural symptoms, although very few clinical studies examining their benefits are available. Similarly, atypical antipsychotic and anti-anxiety/anti-seizure drugs could be prescribed for behavioral symptoms, but their efficacy is not clinically demonstrated in FTD patients. For patients with language variants of FTD, speech therapy is recommended.

**Table 3: Plasma metabolites significantly differentiated between Alzheimer's patients and Frontotemporal Dementia patients.**

| **Abbreviated or Common name** | **IUPAC name (or Traditional name) and HMDB accession number or Subclass or UniProt accession number** |
|---|---|
| L-Glutamic Acid | (2R)-2-aminopentanedioic acid (HMDB03339) |
| C18:2 | Unsaturated fatty acids [FA0103] |
| PC ae (34:3) | Diacylglycerophosphocholines [GP0101] |
| APOE-4 | Apolipoprotein-E (P02649) isoform E4 |

It will be understood that the first method of the invention can be carried out by determining the level of a variable number of metabolites defined in table 1, table 2 or table 3 in the biological sample of the subject under study. For example, the level(s) of at least one, at least two, at least three, at least four, at least five, at least six, at least seven, at least nine, or ten of the markers selected from table 1. Or for example, the level(s) of at least one, at least two, at least three, at least four, at least five, at least six, at least seven, at least nine, or ten of the markers selected from table 2. Or for example, the level(s) of at least one, at least two, at least three, or four of the markers selected from table 3.

The determination of levels of combinations of the metabolic markers may allow greater sensitivity and specificity in the diagnosis of AD or FTD patients.

In a preferred embodiment the metabolic markers of table 1 correspond to the molecules disclosed in table 4 and 7.

In a preferred embodiment the metabolic markers of table 2 correspond to the molecules disclosed in table 5 and 8.

In a preferred embodiment the metabolic markers of table 3 correspond to the molecules disclosed in table 6 and 9.

The term "sample" or "biological sample", as used herein, refers to biological material isolated from a subject. The biological sample may contain any biological material suitable for detecting the desired biomarker and may comprise cellular and/or non-cellular material from the subject. The sample can be isolated from any suitable biological tissue or fluid such as, for example, blood, blood plasma, serum, cerebral spinal fluid (CSF), urine, amniotic fluid, lymph fluids, external secretions of the respiratory, intestinal, genitourinary tracts, tears, saliva, white blood cells. Preferably, the samples used for the determination of the level(s) of the metabolic markers are samples which can be obtained using minimally invasive procedures. In a preferred embodiment, the samples are plasma samples.

The term "level" or "presence", as used herein, refers to the quantity of a biomarker detectable in a sample. Techniques to assay levels of individual biomarkers from test samples are well known to the skilled technician, and the invention is not limited by the means by which the components are assessed. In one embodiment, levels of the individual components of the lipidomic profile are assessed using mass spectroscopy, high performance liquid chromatography (HPLC) and the like. Other methods of assessing levels of the individual components includes isoelectric focusing (IEF) and sandwich ELISA, Immunoturbidimetry, qPCR, northern blot, RNA dot blot, Taq Man, tag based methods such as serial analysis of gene expression (SAGE) including variants such as LongSAGE and SuperSAGE microarrays.

In a preferred embodiment the presence of the Apolipoprotein E isoform 4 (APOE4) is determined by quantitative PCR, preferably Real-Time PCR (RT-PCR) reverse transcription polymerase chain reaction (RT-PCR). In more preferred embodiment, the the presence of allele of the Apolipoprotein E isoform 4 is determined by methods of Immunoturbidimetry as explained in detail in European application number EP15382537 which is incorporate here as reference. The detection can be carried out in individual samples or in tissue microarrays.

The assessment of the levels of the individual components can be expressed as absolute or relative values and may or may not be expressed in relation to another component, a standard an internal standard or another molecule of compound known to be in the sample. If the levels are assessed as relative to a standard or internal standard, the standard may be added to the test sample prior to, during or after sample processing.

To assess levels of the individual components of the subject, a sample is taken from the subject. The sample may or may not processed prior assaying levels of the components of the lipidomic profile. For example, whole blood may be taken from an individual and the blood sample may be processed, e.g., centrifuged, to isolate plasma or serum from the blood. The sample may or may not be stored, e.g., frozen, prior to processing or analysis.

Individual components of the lipidomic profile include but are not limited to phosphatidyl cholines (PC), Lyso PCs, Fatty Acids and Fatty Amides. Specific examples of PCs, Lyso PCs, Fatty Acids and Fatty Amides that can be included as constituents of the lipidomic profile include but are not limited to L-Arginine, L-Glutamic Acid, Ornithine, Aminoadipic acid, Putrescine, Lyso PC a C18:0, PC aa C34:3, PC ae C30:0, PC ae C36:0, L-Alanine, L-Lysine, L-Tryptophan, C18:2, PC aa C34:2, PC aa C42:2, PC ae C38:0, PC ae C34:3. Those of skill in the art will recognize the specific identity of each constituent listed based upon the nomenclature above. For example, metabolite Lyso PC a C18:0 is known as Lysophosphatidylcholine a C18:0, metabolite PC aa C34:2 is known as Phosphatidylcholine with dyacyl residue sum C 34:2), metabolite PC ae C34:3 is known as Phosphatidylcholine with acyl-alkyl residue sum C34:3.

Once the sample has been processed, the first method of the invention involves the determination of the levels of the biomarker in the sample. The expression "determining the levels of the biomarker", as used herein, refers to ascertaining the absolute or relative amount or concentration of the biomarker in the sample. There are many ways to collect quantitative or relational data on biomarkers or metabolites, and the analytical methodology does not affect the utility of metabolite concentrations in assessing a diagnosis. Suitable methods for determining the levels of a given metabolite include, without limitation, refractive index spectroscopy (RI), Ultra-Violet spectroscopy (UV), fluorescent analysis, radiochemical analysis, Infrared spectroscopy (IR), Nuclear Magnetic Resonance spectroscopy (NMR), Light Scattering analysis (LS), Mass Spectrometry, Pyrolysis Mass Spectrometry, Nephelometry, Dispersive Raman Spectroscopy, gas chromatography combined with mass spectroscopy, liquid chromatography combined with mass spectroscopy, supercritical fluid chromatography combined with mass spectroscopy, MALDI combined with mass spectroscopy, ion spray spectroscopy combined with mass spectroscopy, capillary electrophoresis combined with mass spectrometry, NMR combined with mass spectrometry and IR combined with mass spectrometry.

In a particular embodiment, the levels of the metabolic markers are determined by mass spectrometry.

The diagnostic method of the invention comprises comparing the level(s) of the metabolic marker(s) with a reference value.

The term "reference value", as used herein, relates to a predetermined criteria used as a reference for evaluating the values or data obtained from the samples collected from a subject. The reference value or reference level can be an absolute value, a relative value, a value that has an upper or a lower limit, a range of values, an average value, a median value, a mean value, or a value as compared to a particular control or baseline value. A reference value can be based on an individual sample value or can be based on a large number of samples, such as from population of subjects of the chronological age matched group, or based on a pool of samples including or excluding the sample to be tested.

The reference value according to the diagnostic method of the invention can be obtained from one or more subjects who do not suffer from AD or FTD impairment (i.e., healthy control subjects), from subjects suffering from AD or FTD at early stage, non-symptomatic (preclinical stage) or from the same subjects that is been diagnosed or monitoring suffering from AD or FTD but at an earlier time point.

Thus, in a particular embodiment, when the reference value is obtained from a subject not suffering from AD or FTD, increased level(s) of ornithine, putrescine, PC aa C34:3 and/or PC ae C30:0 from table 1 are indicative that the subject suffers from AD; increase levels of L-Alanine, ornithine, C18:2 and/or PC ae C30:0 from table 2 are indicative that the subject suffers from FTD; and/or

decreased level(s) of L-Arginine, L-Glutamic Acid, Alpha-AAA, Lyso PC a C18:0 and/or PC ae C36:0 from table 1 are indicative that the subject suffers from AD; decreased levels of L-Arginine, L-Lysine, L-Tryptophan, PC aa C34:2, PC aa C42:2 and/or PC ae C38:0 from table 2 are indicative that the subject suffers from FTD.

On the other hand increased level(s) of PC ae C34:3 from table 3 in AD patients with respect to FTD patients are indicative that the subject suffers from AD; and /or decreased level(s) of L-Glutamic Acid and/or C18:2 from table 3 in AD with respect to FTD are indicative that the subject suffers from AD.

According to the diagnostic methods of the invention, the level of a metabolic marker is considered "decreased" when the level of said marker in a sample is lower than its reference value. The level of a marker is considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value.

Likewise, in the context of the diagnostic methods of the invention, the level of a marker is considered "increased" when the level of said marker in a sample is higher than its reference value. The level of a marker is considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value.

### Method for determining the efficacy of a therapy for AD and/or FTD

In a second aspect, the invention relates to a method for determining the efficacy of a therapy for AD or FTD comprising determining in a biological sample of a subject suffering from AD or FTD and having been treated with said therapy the level(s) of one or more of the metabolic markers as defined in tables 1 and 2 wherein
- decreased level(s) of ornithine, putrescine, PC aa C34:3 and/or PC ae C30:0 from table 1 in AD with respect to HC or with respect a reference value are indicative that said therapy is effective against AD; decreased level(s) of L-Alanine, ornithine, C18:2 and/or PC ae C30:0 from table 2 in FTD with respect to HC or with respect a reference value are indicative that said therapy is effective against FTD; and /or
- increased level(s) of L-Arginine, L-Glutamic Acid, Alpha-AAA, LysoPC a C18:0 and/or PC ae C36:0 from table 1 in AD with respect to HC or with respect a reference value are indicative that said therapy is effective against AD; increased level(s) of L-Arginine, L-Lysine, L-Tryptophan, PC aa C34:2, PC aa C42:2 and/or PC ae C38:0 from table 2 in FTD with respect to HC or with respect a reference value are indicative that said therapy is effective against FTD.

The terms "AD","FTD", "subject", "metabolic marker", "biological sample", "level", "decreased" and "increased" have been defined in connection with the first diagnostic method of the invention. The metabolic markers defined in Tables 1 and 2, as well as the techniques for determining the level or the presence of these markers, have also been defined in connection to the diagnostic methods of the invention. The particular and preferred embodiments of the diagnostic methods of the invention regarding these terms are also applicable to the second method of the invention.

The term "subject suffering from AD or FTD" or "AD or FTD patient", as used herein, refers to a subject who has been diagnosed with AD or FTD.

The term "therapy for AD or FTD" as used herein, refers to the attempted remediation of a health problem, usually following a diagnosis, or to prevention of the appearance of a health problem. As such, it is not necessarily a cure, i.e. a complete reversion of a disease. Said therapy may or may not be known to have a positive effect on a particular disease. This term includes both therapeutic treatment and prophylactic or preventative measures, in which the object is to prevent or stop (reduce) an undesired physiological change or disorder, such as, AD or FTD. For the purpose of this invention, beneficial or desired clinical results include, without limitation, relieving symptoms, reducing the spread of the disease, stabilizing pathological state (specifically not worsening), slowing down or stopping the progression of the disease, improving or mitigating the pathological state and remission (both partial and complete), both detectable and undetectable. It can also involve prolonging survival, disease free survival and symptom free survival, in comparison with the expected survival if treatment is not received. Those subjects needing treatment include those subjects already suffering the condition or disorder, as well as those with the tendency to suffer the condition or disorder or those in which the condition or disorder must be prevented. For illustrative non-limitative examples of AD or FTD therapies, please refer to the specification above for additional treatments and for the methods to confirm the clinical diagnosis of any of these diseases.

In a particular embodiment, the biological samples are plasma samples.

In a particular embodiment, the second diagnostic method of the invention comprises determining the levels of L-Arginine.

In a particular embodiment, the second diagnostic method of the invention comprises determining the levels of L-Glutamic Acid.

In a particular embodiment, the second diagnostic method of the invention comprises determining the levels of ornithine.

In a particular embodiment, the second diagnostic method of the invention comprises determining the levels of putrescine.

In a particular embodiment, the second diagnostic method of the invention comprises determining the levels of Lyso PC a C18:0.

In a particular embodiment, the second diagnostic method of the invention comprises determining the levels of PC aa C34:3.

In a particular embodiment, the second diagnostic method of the invention comprises determining the levels of PC ae C30:0.

In a particular embodiment, the second diagnostic method of the invention comprises determining the levels of PC ae C36:0.

In a particular embodiment, the second diagnostic method of the invention comprises determining the levels of L-Alanine.

In a particular embodiment, the second diagnostic method of the invention comprises determining the levels of L-Lysine.

In a particular embodiment, the second diagnostic method of the invention comprises determining the levels of L-Tryptophan.

In a particular embodiment, the second diagnostic method of the invention comprises determining the levels of C18:2.

In a particular embodiment, the second diagnostic method of the invention comprises determining the levels of PC aa C34:2.

In a particular embodiment, the second diagnostic method of the invention comprises determining the levels of PC aa C42:2.

In a particular embodiment, the second diagnostic method of the invention comprises determining the levels of PC ae C38:0.

In a particular embodiment, the second method of the invention comprises determining the levels of at least two, at least three, at least four, at least five, at least six, at least seven, at least eight, at least nine, or ten of the markers selected from table 1 and/or table 2.

In a preferred embodiment the metabolic markers of Table 1 correspond to the molecules disclosed in Tables 4 and 7 and the metabolic markers of Table 2 correspond to the molecules disclosed in Tables 5 and 8.

The second method of the invention involves comparing the levels of one or more metabolic markers in a sample from a subject with a reference value. In a particular embodiment, the reference value is determined in a sample from the same subject before being contacted with the therapy. In another particular embodiment, the reference is determined in a sample from one or more subjects suffering from AD or FTD left untreated or having been treated with a control therapy. The term "control therapy", as used herein, refers to a therapy with no effect on AD or FTD. Preferably, the control therapy has the same excipient, carrier or vehicle which is used in the therapy whose efficacy for the treatment of AD or FTD is to be assessed.

In a particular embodiment, the determination of the level of the one or more metabolic markers is carried out by mass spectrometry.

### Method for the identification of compounds suitable for the treatment of AD and/or FTD

In a third aspect, the invention relates to a method for the identification of compounds for AD or FTD therapy comprising determining in a biological sample of a subject having been treated with said test compound the levels of at least one metabolic marker or markers described in table 1 and table 2 wherein
- decreased level(s) of ornithine, putrescine, PC aa C34:3 and/or PC ae C30:0 from table 1 in AD with respect to HC or with respect a reference value are indicative that the compound is effective for the treatment of AD; decreased level(s) of L-Alanine, ornithine, C18:2 and/or PC ae C30:0 from table 2 in FTD with respect to HC or with respect a reference value are indicative that the compound is effective for the treatment of FTD; and /or
- increased level(s) of L-Arginine, L-Glutamic Acid, Alpha-AAA, LysoPC a C18:0 and/or PC ae C36:0 from table 1 in AD with respect to HC or with respect a reference value are indicative that the compound is effective for the treatment of AD,; increased level(s) of L-Arginine, L-Lysine, L-Tryptophan, PC aa C34:2, PC aa C42:2 and/or PC ae C38:0 from table 2 in FTD with respect to HC or with respect a reference value are indicative that the compound is effective for the treatment of FTD;
wherein preferably the method is performed *in vitro.* It is noted that the third aspect includes the isolation and the optional purification of the test compound.

The terms "AD", "FTD", "biological sample", "level", "metabolic marker", "increased" and "decreased" have been defined in connection with the first diagnostic methods of the invention. The metabolic markers defined in Tables 1 and 2, as well as the techniques for determining the level of these markers, have also been defined in connection to the diagnostic methods of the invention. The particular and preferred embodiments of the diagnostic methods of the invention regarding these terms are also applicable to the third method of the invention.

The metabolic markers defined in Tables 1 and 2, as well as the techniques for determining the level of these markers, have also been defined in connection to the diagnostic method of the invention. The particular and preferred embodiments of the diagnostic method of the invention regarding these terms are also applicable to the third method of the invention.

The term "subject suffering from AD or FTD" has been defined in connection with the first diagnostic method of the invention.

Examples of suitable animals for use in the third method of the invention include, but are not limited to, mice, rats, rabbits, monkeys, guinea pigs, dogs and cats. In accordance with this embodiment, the test compound or a control compound is administered (e.g., orally, rectally or parenterally such as intraperitoneally or intravenously) to a suitable animal and the effect on the levels of one or more of the metabolites shown in tables 1 or 2 is determined. Examples of agents include, but are not limited to, nucleic acids (e.g., DNA and RNA), carbohydrates, lipids, proteins, peptides, peptidomimetics, small molecules and other drugs. Agents can be obtained using any of the numerous approaches in combinatorial library methods known in the art. Test compounds further include, for example, antibodies (e.g., polyclonal, monoclonal, humanized, anti-idiotypic, chimeric, and single chain antibodies as well as Fab, F(ab') 2, Fab expression library fragments, and epitope-binding fragments of antibodies). Further, agents or libraries of compounds may be presented, for example, in solution, on beads, chips, bacteria, spores, plasmids or phage.

If the compound is a low-molecular weight compound, then this can be generated by various methods known to the art, preferably synthetically, in particular by combinatorial chemistry, or by biochemical methods, in particular by recombinant expression or purification from biological probes. The compound is of low molecular weight ("small molecules") or the library is composed of molecules with low molecular weight ("small molecule library"). A "small molecule" is defined as a complex collection of compounds, which are produced in a non-biological way, which means which are not produced by recombinant expression, like for instance most protein or peptide libraries. "Small molecules" can be generated by various methods known to the art, but are preferably produced by synthetically, more preferably by combinatory chemistry, to generate a compound library with a maximum chemical diversity within the constraints of predicted attractive drug characteristics. If the compound to be assayed for its suitability for the treatment of AD and/or FTD is a peptide or a peptide library, then these can be generated by various methods known to the art for their use as candidate compounds, but they are preferably produced by biochemical methods, more preferably by recombinant expression in prokaryotic or eukaryotic cells.

The compound to be tested for its suitability for the therapy of AD and/or FTD can be formulated with a pharmaceutically acceptable carrier to produce a pharmaceutical composition, which can be administered to a human or other animal. A pharmaceutically-acceptable carrier can be, for example, water, sodium phosphate buffer, phosphate-buffered saline, normal saline or Ringer's solution or other physiologically-buffered saline, or other solvent or vehicle such as a glycol, glycerol, an oil such as olive oil or an injectable organic ester. A pharmaceutically acceptable carrier can also contain physiologically acceptable compounds that act, for example, to stabilize or increase the absorption of the modulatory compound. One skilled in the art would know that the choice of a pharmaceutically acceptable carrier, including a physiologically acceptable compound, depends, for example, on the route of administration of the composition.

In a particular embodiment, the third diagnostic method of the invention comprises determining the levels of L-Arginine.

In a particular embodiment, the third diagnostic method of the invention comprises determining the levels of L-Glutamic Acid.

In a particular embodiment, the third diagnostic method of the invention comprises determining the levels of Ornithine.

In a particular embodiment, the third diagnostic method of the invention comprises determining the levels of Putrescine.

In a particular embodiment, the third diagnostic method of the invention comprises determining the levels of Lyso PC a C18:0.

In a particular embodiment, the third diagnostic method of the invention comprises determining the levels of PC aa C34:3.

In a particular embodiment, the third diagnostic method of the invention comprises determining the levels of PC ae C30:0.

In a particular embodiment, the third diagnostic method of the invention comprises determining the levels of PC ae C36:0.

In a particular embodiment, the third diagnostic method of the invention comprises determining the levels of L-Alanine.

In a particular embodiment, the third diagnostic method of the invention comprises determining the levels of L-Lysine.

In a particular embodiment, the third diagnostic method of the invention comprises determining the levels of L-Tryptophan.

In a particular embodiment, the third diagnostic method of the invention comprises determining the levels of C18:2.

In a particular embodiment, the third diagnostic method of the invention comprises determining the levels of PC aa C34:2.

In a particular embodiment, the third diagnostic method of the invention comprises determining the levels of PC aa C42:2.

In a particular embodiment, the third diagnostic method of the invention comprises determining the levels of PC ae C38:0.

In a particular embodiment, the third method of the invention comprises determining the levels of at least two, at least three, at least four, at least five, at least six, at least seven, at least eight, at least nine, or ten of the markers selected from table 1 and table 2.

In a preferred embodiment the metabolic markers of Table 1 correspond to the molecules disclosed in Tables 4 and 7 and the metabolic markers of Table 2 correspond to the molecules disclosed in Tables 5 and 8.

The third method of the invention involves comparing the levels of one or more metabolic markers in a sample from the subject with a reference value. In a particular embodiment, the reference value is determined in a sample from the same subject before being contacted with a candidate compound. In another particular embodiment, the reference is determined in a sample from one or more subjects suffering from AD or FTD left untreated or having been treated with a control therapy, preferably the same excipient, carrier or vehicle which is used with the candidate compound.

In a particular embodiment, the determination of the level of the one or more metabolic markers is carried out by mass spectrometry.

### Method for monitoring the progression of AD and/or FTD

In a fourth aspect, the invention relates to a method for monitoring the progression of a subject suffering from AD or FTD comprising determining in a biological sample of a subject, over the course of a therapy or not, the levels of at least one metabolic marker or markers described in table 1 and table 2 wherein
- increased level(s) of Ornithine, Putrescine, PC aa C34:3 and/or PC ae C30:0 from table 1 with respect to a reference value determined in a sample from the same subject at an earlier time point are indicative that AD condition is progressing; increased level(s) of L-Alanine, Ornithine, C18:2 and/or PC ae C30:0 from table 2 with respect to a reference value determined in a sample from the same subject at an earlier time point are indicative that FTD condition is progressing; and/or
- decreased level(s) of L-Arginine, L-Glutamic Acid, Alpha-AAA, Lyso PC a C18:0 and/or PC ae C36:0 from table 1 with respect to a reference value determined in a sample from the same subject at an earlier time point are indicative that AD condition is progressing; decreased level(s) of L-Arginine, L-Lysine, L-Tryptophan, PC aa C34:2, PC aa C42:2 and/or PC ae C38:0 from table 2 with respect to a reference value determined in a sample from the same subject at an earlier time point are indicative that FTD condition is progressing.

The terms "AD", "FTD", "subject", "metabolic marker", "biological sample", "level", "decreased" and "increased" have been defined in connection with the first diagnostic method of the invention. The metabolic markers defined in Tables 1 and 2, as well as the techniques for determining the level of these markers, have also been defined in connection to the diagnostic methods of the invention. The particular and preferred embodiments of the diagnostic methods of the invention regarding these terms are also applicable to the fourth method of the invention

The term "subject suffering from AD or FTD" or "AD or FTD patient" as used herein, refers to a subject who has been diagnosed with AD or FTD.

The term "monitoring the progression", as used herein, refers to the determination of the evolution of the disease in a subject diagnosed with AD or FTD, i.e., whether the AD or FTD is worsening or whether it is ameliorating.

The term "progression in the AD or FTD", as used herein, is understood as a worsening of the disease, i.e., that the disease is progressing to a later stage with respect to a stage at an earlier time point measured. In a particular embodiment, a progression in the AD or FTD means a progression from AD or FTD in a preclinical stage (non- symptomatic subjects) to AD or FTD with clinical symptoms (symptomatic subjects).

In a particular embodiment, the biological samples are plasma samples.

In a particular embodiment, the fourth diagnostic method of the invention comprises determining the levels of L-Arginine.

In a particular embodiment, the fourth diagnostic method of the invention comprises determining the levels of L-Glutamic Acid.

In a particular embodiment, the fourth diagnostic method of the invention comprises determining the levels of Ornithine.

In a particular embodiment, the fourth diagnostic method of the invention comprises determining the levels of Putrescine.

In a particular embodiment, the fourth diagnostic method of the invention comprises determining the levels of Lyso PC a C18:0.

In a particular embodiment, the fourth diagnostic method of the invention comprises determining the levels of PC aa C34:3.

In a particular embodiment, the fourth diagnostic method of the invention comprises determining the levels of PC ae C30:0.

In a particular embodiment, the fourth diagnostic method of the invention comprises determining the levels of PC ae C36:0.

In a particular embodiment, the fourth diagnostic method of the invention comprises determining the levels of L-Alanine.

In a particular embodiment, the fourth diagnostic method of the invention comprises determining the levels of L-Lysine.

In a particular embodiment, the fourth diagnostic method of the invention comprises determining the levels of L-Tryptophan.

In a particular embodiment, the fourth diagnostic method of the invention comprises determining the levels of C18:2.

In a particular embodiment, the fourth diagnostic method of the invention comprises determining the levels of PC aa C34:2.

In a particular embodiment, the fourth diagnostic method of the invention comprises determining the levels of PC aa C42:2.

In a particular embodiment, the fourth diagnostic method of the invention comprises determining the levels of PC ae C38:0.

In a particular embodiment, the fourth method of the invention comprises determining the levels of at least two, at least three, at least four, at least five, at least six, at least seven, at least eight, at least nine, or ten of the markers selected from table 1 and table 2.

In a preferred embodiment the metabolic markers of Table 1 correspond to the molecules disclosed in Tables 4 and 7 and the metabolic markers of Table 2 correspond to the molecules disclosed in Tables 5 and 8.

The fourth method of the invention involves comparing the levels of one or more metabolic markers in a sample from a subject with a reference value, i.e., the levels of the same marker or markers, determined in a sample from said subject at an earlier time point. In a particular embodiment, the level of the marker in a sample is compared with the level of said marker in the same type of sample.

In a particular embodiment, the determination of the level of the one or more metabolic markers is carried out by mass spectrometry. In particular, by using a kit suitable for mass spectrometry assay preparation. Such kit should preferably deliver the widest range of metabolomic information available from a single targeted assay, covering a large number of key metabolites from main metabolic pathways. This kit should thus quantitatively analyze a large number of metabolites that have already been identified herein as part of key biochemical pathways, providing fundamental data to link changes in the metabolome to biological events. Preferably, such kit should preferably comprise at least one, preferably all, of the following components; a kit's plate, a silicone mat cover for the plate, solvents preferably in sealed glass ampoules, quality controls, standards, a deep well capture plate, a memory stick having a software to link changes in the metabolome to biological events and a user manual.

Yet another aspect of the present invention includes a kit for a diagnosis of AD or FTD or for the differential diagnosis of AD and FTD, comprising: biomarker detecting reagents for determining a differential expression level of at least one of the markers identified in table 1 or in table 2 or in table 3 or any combination thereof, preferably comprising biomarker detecting reagents for determining a differential expression level of all of the biomarkers identified in table 1 or in table 2 or in table 3.

In one preferred embodiment of this aspect of the invention, the kit further comprises instructions for use in diagnosing risk for AD or FTD or for the differential diagnosis of AD and FTD, wherein the instruction comprise step-by-step directions to compare the expression level of at least one, preferably all, of the markers identified in table 1 or in table 2 or in table 3, when measuring the expression of a sample obtained from a subject suspected of having AD or FTD with the expression level of a sample obtained from a normal subject, wherein the normal subject is a healthy subject not suffering from AD or FTD, or with a reference value. In another aspect, the kit further comprises tools, vessels and reagents necessary to obtain samples from a subject selected from the group consisting of one or more biological fluids, a plasma sample, a serum sample, a blood sample, a tissue sample, or a faecal sample, preferably a plasma sample.

Yet another aspect of the present invention includes a computer program suitable for implementing any of the methods of the present invention. In addition, a device comprising the above mentioned computer program also forms part of the present invention as well as its use for the diagnosis of AD or FTD in a human subject.

In particular this aspect of the invention refers to the *in vitro* use of a diagnostic kit or device comprising reagents capable of measuring the levels of at least one metabolic marker selected from the list of biomarkers of table 1 or table 2 or table 3 or any combination thereof in a plasma sample, for distinguish Alzheimer's patient subjects or FTD patient subjects from subjects not suffering from Alzheimer's disease or from FTD. Preferably said Kit comprises reagents capable of measuring the levels of all of the metabolic markers identified in table 1 or in table 2 or in table 3.

Another preferred embodiment of the present invention refers to the *in vitro* use of a kit or device comprising reagents capable of measuring the levels of at least one metabolic marker selected from the list of biomarkers of table 1 or table 2 or any combination thereof in a plasma sample, for determining the efficacy of a therapy for AD or FTD in a subject according to the second aspect of the invention. Preferably said Kit comprises reagents capable of measuring the levels of all of the metabolic markers identified in table 1 or in table 2.

Another preferred embodiment of the present invention refers to the *in vitro* use of a kit or device comprising reagents capable of measuring the levels of at least one metabolic marker selected from the list of biomarkers of table 1 or table 2 or any combination thereof in a plasma sample, for monitoring the progression of AD or FTD in a subject in a subject according to the third aspect of the invention. Preferably said Kit comprises reagents capable of measuring the levels of all of the metabolic markers identified in table 1 or in table 2.

Another preferred embodiment of the present invention refers to the *in vitro* use of a kit or device comprising reagents capable of measuring the levels of at least one metabolic marker selected from the list of biomarkers of table 1 or table 2 or any combination thereof in a plasma sample, for monitoring the progression of AD or FTD in a subject in a subject according to the fourth aspect of the invention. Preferably said Kit comprises reagents capable of measuring the levels of all of the metabolic markers identified in table 1 or in table 2.

The assignment of a patient into a specific group of patients, such as patients with AD or FTD, by any of the methods of the invention can be done by a computer program, preferably, after introducing the data into said program. Thus, in another preferred embodiment, the step of assigning a patient into a specific group of patients, such as patients with AD or FTD, according to any of the methods described in the present specification, is a computer implemented step wherein the data obtained in the previous steps of the method are inserted in a computer program and the program assigns the patient into one of the groups of patients.

This differentiation between patients may help to determine the best treatment aimed to reduce the symptomatology of AD or FTD.

The present invention is further illustrated by the following examples which merely illustrate the invention and do not limit the same.

### EXAMPLE

### Materials and methods

### Patients data

A total of 340 volunteers participated in this multicenter international study (9 Spanish, 2 French and 4 Belgian hospitals/centers were involved) the participants were classified as follow:

### Inclusion Criteria:

| **Healthy Controls = 154 patients** | |
|---|---|
| **Sex (M/F)** | **YES** |
| **Age** | Between 50-80 years old |
| **Neuropsychological screening test result** | Negative |
| **Analysis of markers in cerebrospinal fluid (at least 24 months before the recruitment)** | Negative for: β-amyloid TAU protein p-TAU |

| **Alzheimer Disease Patients = 118 patients** | |
|---|---|
| **Sex (M/F)** | YES |
| **Age** | Between 50-80 years old |
| **Clinic Criteria for AD (Criteria of NIA-AA, Alzheimer Dementia, 2011).** | Positive |
| **Analysis of markers in cerebrospinal fluid (at least 36 months before the recruitment)** | Positive for: β-Amyloid TAU proteinp-TAU |

| **Frontotemporal Dementia Patients = 68 patients** | |
|---|---|
| **Sex (M/F)** | YES |
| **Age** | Between 50-80 years old |
| **Clinic Criteria for FTD (FTD clinic diagnostic criteria based on Neary, 1998, Rascovsky, 2011).** | Positive |
| **Image Test (Magnetic Resonance, PET o SPECT)** | Frontal or frontotemporal lobes with progressive cortical atrophy YES |
| ***Optional but recommended:* Analysis of markers in cerebrospinal fluid (at least 36 months before the recruitment)** | Negative for β-amyloid Negative or Positive for: TAU protein & p-TAU |

### Exclusion Criteria:

- Severe or acute systemic disease that may make it difficult to study or follow the participant:
   - Advanced liver or kidney disease
   - Disseminated neoplastic disease
- Addiction to alcohol or other drugs in the last 2 years according to DSM-IV criteria, except for the use of nicotine, which is permitted
- Down syndrome
- Moderate or severe cranioencephalic trauma
- CNS infections (HIV, lupus, borrelia, herpes simplex, suspected Creutzfeldt Jakob disease)
- Endocrine disorders (thyroid disorders)
- Nutritional deficits (vitamin B12, folic acid)
- Clinical history of stroke in the previous 3 months or evidence by neuroimaging of clinically significant cardiovascular disease.
- Neurological diseases (demyelinating diseases, Parkinson's disease, Huntington's disease, normotensive hydrocephalus, subdural hematoma, brain tumors)
- Major psychiatric disorder (major depression or psychosis)
- Patients who do not have a clearly defined clinical diagnosis according to the study groups (Inclusion / Exclusion criteria)
- Disease that in the opinion of the investigator or the promoter may have a potentially significant influence and therefore.

### Sampling

Patients recruited were fasted (minimum of 8 hours), and then the analytical sample was extracted. Two tubes of whole blood (1 Vacutainer tube with EDTA, one tube for Vacutainer serum without anticoagulant, in total a maximum volume of 20 mL) were obtained, which were processed within 2 hours (room temperature). Once the different fractions were obtained they were stored at -80 ° C until processed.

### Sample Processing

1. 10 mL of whole blood were collected in EDTA tube (purple cap) previously labelled with patient code.
2. Samples were centrifuged at 4°C, 1.700 x g 10min in a centrifuge with progressive braking (dropping from 1700 x g to 0 in approximately 30 seconds). Sudden movements after centrifugation were avoided in order to avoid mixing the different blood fractions.
3. All possible 1 mL aliquots of plasma were collected (usually 4) in 1.5 mL previously labelled Eppendorf tubes. Special care was taken at this point to avoid carrying any Buffy coat (the BC is a cell-rich membrane layer between the plasma and the haematies concentrate).
4. All 1 mL aliquots of plasma extracted in the previous step were centrifuged for 10 minutes at 13000 rpm. Platelet-free plasma (PFP) were obtained.
5. Complete supernatant (PFP) was collected in 5 mL Eppendorf tube, previously labelled with the labels provided by Raman Health Technologies and immediately stored at 80°C.
6. Buffy Coat (BC) was recovered by inserting the pipette to the level of the white membranous interphase layer and with extreme care, the entire phase of white cells was collected, pulling approximately 0.5 mL of plasma from above and 0.5 mL of haematies concentrate from below, obtaining an aliquot of 1 mL of BC. This fraction was dispensed into a 5mL Eppendorf tube previously labelled with the labels provided by Raman Health Technologies and immediately stored at -80°C.
7. 10 mL of whole blood in 1 previously labeled serum tube will be drawn
8. Centrifuge 10 min at 1000 x g in a centrifuge that has brake. Avoid sudden movements after centrifuging the tube so that different fractions of blood do not mix. Transfer the serum to Eppendorf tubes.

Analysis of APOE4: APOE genotype was carried out using Real Time PCR. An individual was assigned with a value of 1, if the genotype revealed the presence of one ε4 allele or two ε4 alleles of the *APOE* gene, or 0 if the genotype revealed the absence of the ε4 allele.

### Metabolite analysis:

A total of 188 metabolites were analyzed in plasma samples from patients and controls using the validated quantitative kit *Absolute*® *IDQ p180 kit* (Biocrates, Innsbruck Austria). This kit performed a targeted metabolite profiling by electrospray ionization (ESI) tandem mass spectrometry (MS/MS) and it was performed on a fee-for-service basis on its quantitative metabolomics platform at Biocrates Life Sciences AG, Austria. The experimental metabolomics measurement technique is described in detail by patent US 2007/0004044 (http://www.freepatentsonline.com/20070004044.html). This kit comprises 40 acylcarnitines, 42 amino acids and biogenic amines, 15 sphingolipids, 90 glycerophospholipids and sum hexoses.

### Statistical Analysis:

An iterative model building approach was applied to identify discriminative signatures:
1) Univariate analysis of all metabolites for each signature (AD vs HC, FTD vs HC and AD vs FTD)
2) Creation of a list of key discriminative metabolites (significant at the 5% level)
3) Cross-validation by stepwise logistic regression
4) Variables which occur in at least 25% of cross-validations were entered into a logistic regression to obtain the final discriminative signatures.

The univariate analysis was performed for each comparison group to investigate the variables that had a significant association at the 5% level with an individual's classification. To do so, a simple logistic regression was performed, modelling the diagnosis as a binary response with 1 for a case, and 0 as a control, against each variable of interest. Variables were assessed using the Wald test to determine the significance of each variable. The models were also tested for convergence, to identify issues such as quasi-complete or complete separation of data points which could cause further modelling issues downstream.

Following the univariate analysis, a list of variables from the relevant parameter groups which had a significant association at the 5% level with an individual's classification, and did not present any convergence issues in the univariate analyses were retained. Whilst the association of the demographic variables, age and sex, may be significant, these variables were excluded from subsequent analyses. As a result, only the markers of interest were included in the final classification models built using a process of stepwise selection within a series of cross validations.

Cross-validation was utilized as a robust method to determine the combination of variables that are significantly associated with an individual's classification, independent of the sample of data being analyzed. The cross-validations consisted of 100 random samples using a 70% sample for the training dataset, whilst the remaining 30% sample of data was used for validation.

Within each cross-validation, a logistic regression assuming stepwise selection, and using an entry criteria equalling p<0.05 and stay criteria equalling p<0.1, was used to determine a final model for each iteration. The frequency of times a variable was included within the final models was recorded. Those variables found within at least 25% of derived models were then included within a fixed logistic regression model without selection to determine the combination of markers to be included in the final model for classification. Again, the performance of the final marker combination was further evaluated using the conservative method of cross-validation.

Evaluation of the final models: the second round of cross-validation enabled the determination of accuracy rates based upon the classification rules containing the final marker combination. Two types of accuracy rates were observed, namely the area under the ROC curve (AUC) and 1-misclassifcation rate.

The cross-validation performed logistic regression without selection on the most frequent variables chosen as above. Again, the cross-validations consisted of 100 random samples using a 70% sample for the training dataset, whilst the remaining 30% sample of data was used for validation. Doing so enabled the average, standard deviation and 95% confidence intervals of these accuracy rates to be determined for each diagnosis comparison. The ROC curves and parameter estimates were provided based on the logistic regression model with an AUC closest to the average AUC calculated through cross-validation.

| **Comparison** | **AUC** | | **1-Misclassification Rate** | |
|---|---|---|---|---|
| | **Mean** | **S.D.** | **Mean** | **S.D.** |
| Diagnosis AD vs HC | 0.96 | 0.023 | 0.88 | 0.041 |
| Diagnosis FTD vs AD | 0.79 | 0.049 | 0.75 | 0.054 |
| Diagnosis FTD vs HC | 0.92 | 0.031 | 0.85 | 0.036 |

Tables 4 show the specific p-value (P> chi-square) for the increased and decreased plasma metabolites in the AD patients vs. HC.

Tables 5 show the specific p-value ((P> chi-square) for the increased and decreased plasma metabolites in FTD patients vs. HC.

Tables 6 show the specific p-value ((P> chi-square) for the increased and decreased plasma metabolites in AD patients vs. FTD patients.

Tables 7, 8, 9, show the values of the cross-validation method that undergone these selected metabolites.

**Table 4. p-value (Pr>Chi-Square) for increased and decreased plasma metabolites in AD vs. HC.**

| | | | **AD vs. HC** | |
|---|---|---|---|---|
| **Abbreviated or Common name** | **IUPAC name (or Traditional name) and HMDB accession number or Subclass or UniProt accession number** | **Subclass** | **log2(fold change)** | **Pr> Chi-Square (p-value)** |
| L-Arginine | (2S)-2-amino-5-carbamimidamidopentanoic acid (HMDB00517) | Amino Acids and Biogenic Amines | 0,872741 | 0,000104 119 |
| L-Glutamic Acid | (2R)-2-aminopentanedioic acid (HMDB03339) | Amino Acids and Biogenic Amines | 0,754941 | 1,11933E05 |
| Ornithine | (2S)-2,5-diaminopentanoic acid (HMDB00214) | Amino Acids and Biogenic Amines | 1,189719 | 2,52628E-05 |
| Aminoadipic acid | 2-aminohexanedioic acid(HMDB00510) | Amino Acids and Biogenic Amines | 0,832320 | 0,005525 754 |
| Putrescine | Butane-1,4-diamine (HMDB01414) | Amino Acids and Biogenic Amines | 1,534844 | 0,000226 014 |
| Lyso PC a C18:0 | 1-octadecanoyllysolecithin (HMDB10384) | Glycerophospholipid s | 0,684581 | 7,93946E-09 |
| PC aa C34:3 | Diacylglycerophosphocholines [GP0101] | Glycerophospholipid s | 1,240971 | 0,000130 191 |
| PC ae C30:0 | Diacylglycerophosphocholines [GP0101] | Glycerophospholipid s | 1,092642 | 0,041004 466 |
| PC ae C36:0 | Diacylglycerophosphocholines [GP0101] | Glycerophospholipid s | 0,843847 | 0,001450 13 |
| APOE4 | Apolipoprotein-E (P02649) isoform E4 | NA | NA | 1,85433E-10 |

**Table 5. p-value (Pr>Chi-Square) for increased and decreased plasma metabolites in FTD patients vs. HC.**

| | | | **FTD vs. HC** | |
|---|---|---|---|---|
| **Abbreviated or Common name** | **IUPAC name (or Traditional name) and HMDB accession number or Subclass or UniProt accession number** | **Subclass** | **log2(fold change)** | **Pr>Chi-Square (p-value)** |
| L-Alanine | (2S)-2-aminopropanoic acid (HMDB00161) | Amino Acids and Biogenic Amines | 1,126803 | 0,005118225 |
| L-Arginine | (2S)-2-amino-5-carbamimidamidopentanoic acid (HMDB00517) | Amino Acids and Biogenic Amines | 0,789133 | 3,80867E-07 |
| L-Lysine | (2S)-2,6-diaminohexanoic acid (HMDB00182) | Amino Acids and Biogenic Amines | 0,904460 | 0,010779244 |
| Ornithine | (2S)-2,5-diaminopentanoic acid (HMDB00214) | Amino Acids and Biogenic Amines | 1,204673 | 0,000110072 |
| L-Tryptophan | 2S)-2-amino-3-(1H-indol-3-yl)propanoic acid (HMDB009299) | Amino Acids and Biogenic Amines | 0,888733 | 0,000584318 |
| C18:2 | Unsaturated fatty acids [FA0103] | Acylcarnitine | 1,375094 | 6,37509E-08 |
| PC aa C34:2 | Diacylglycerophosphocholines [GP0101] | Glycerophospholipids | 0,879185 | 0,000706961 |
| PC aa C42:2 | Diacylglycerophosphocholines [GP0101] | Glycerophospholipids | 0,863840 | 0,005940535 |
| PC(30:0) | Diacylglycerophosphocholines [GP0101 | Glycerophospholipids | 1,250093 | 0,000305963 |
| PC(38:0) | Diacylglycerophosphocholines [GP0101] | Glycerophospholipids | 0,900386 | 0,047282952 |

**Table 6. p-value (Pr>Chi-Square) for increased and decreased plasma metabolites in AD patients vs. FTD.**

| | | | **AD vs**. **FTD** | |
|---|---|---|---|---|
| **Abbreviated or Common name** | **IUPAC name (or Traditional name) and HMDB accession number or Subclass or UniProt accession number** | **Subclass** | **log2(fold change)** | **Pr>ChiSquare (p-value)** |
| L-Glutamic Acid | (2R)-2-aminopentanedioic acid (HMDB03339) | Amino Acids and Biogenic Amines | 0,751913 | 0,000756084 |
| C18:2 | Unsaturated fatty acids [FA0103] | Acylcarnitine | 0,863882 | 0,015715192 |
| PC ae C34:3 | Diacylglycerophosphocholines [GP0101] | Glycerophospholipids | 1,374593 | 0,000310225 |
| APOE-4 | Apolipoprotein-E (P02649) isoform E4 | NA | NA | 8,50213E-07 |

**Table 7. Cross validation of significant metabolites from table 1 and 4: "Frequency of stepwise" shows how many times each metabolite has been included in predictive combinations, the number of those repetitions and the percentage in which the metabolite has been included**

| | | **Frequency of Stepwise** | | |
|---|---|---|---|---|
| **Abbreviated or Common name** | **IUPAC name (or Traditional name) and HMDB accession number or Subclass or UniProt accession number** | **Times Included** | **Number of Iterations** | **Percentage (%)** |
| L-Arginine | (2S)-2-amino-5-carbamimidamidopentanoic acid (HMDB00517) | 22 | 83 | 26,51 |
| L-Glutamic Acid | (2R)-2-aminopentanedioic acid (HMDB03339) | 33 | 83 | 39,76 |
| Ornithine | (2S)-2,5-diaminopentanoic acid (HMDB00214) | 26 | 83 | 31,33 |
| Aminoadipic acid | 2-aminohexanedioic acid(HMDB00510) | 34 | 83 | 40,96 |
| Putrescine | Butane-1,4-diamine (HMDB01414) | 55 | 83 | 66,27 |
| LysoPC(18:0) | 1-octadecanoyl lysolecithin (HMDB10384) | 52 | 83 | 62,65 |
| PC(34:3) | Diacylglycerophosphocholines [GP0101] | 41 | 83 | 49,4 |
| PC(30:0) | Diacylglycerophosphocholines [GP0101] | 71 | 83 | 85,54 |
| PC(36:0) | Diacylglycerophosphocholines [GP0101] | 41 | 83 | 49,4 |
| APOE4 | Apolipoprotein-E (P02649) isoform E4 | 82 | 83 | 98,8 |

**Table 8. Cross validation of significant metabolites from table 2 and 5: "Frequency of stepwise" shows how many times each metabolite has been included in predictive combinations, the number of those repetitions and the percentage in which the metabolite has been included.**

| | | **Frequency of Stepwise** | | |
|---|---|---|---|---|
| **Abbreviated or Common name** | **IUPAC name (or Traditional name) and HMDB accession number or Subclass or UniProt accession number** | **Times Included** | **Number of Iterations** | **Percentage (%)** |
| L-Alanine | (2S)-2-aminopropanoic acid (HMDB00161) | 27 | 100 | 27 |
| L-Arginine | (2S)-2-amino-5-carbamimidamidopentanoic acid (HMDB00517) | 49 | 100 | 49 |
| L-Lysine | (2S)-2,6-diaminohexanoic acid (HMDB00182) | 29 | 100 | 29 |
| Ornithine | (2S)-2,5-diaminopentanoic acid (HMDB00214) | 76 | 100 | 76 |
| L-Tryptophan | 2S)-2-amino-3-(1H-indol-3-yl)propanoic acid (HMDB009299) | 57 | 100 | 57 |
| C18:2 | Unsaturated fatty acids [FA0103] | 95 | 100 | 95 |
| PC(34:2) | Diacylglycerophosphocholines [GP0101] | 51 | 100 | 51 |
| PC(42:2) | Diacylglycerophosphocholines [GP0101] | 90 | 100 | 90 |
| PC(30:0) | Diacylglycerophosphocholines [GP0101 | 70 | 100 | 70 |
| PC(38:0) | Diacylglycerophosphocholines [GP0101] | 57 | 100 | 57 |

**Table 9. Cross validation of significant metabolites from table 3 and 6: "Frequency of stepwise" shows how many times each metabolite has been included in predictive combinations, the number of those repetitions and the percentage in which the metabolite has been included.**

| | | **Frequency of Stepwise** | | |
|---|---|---|---|---|
| **Abbreviated or Common name** | **IUPAC name (or Traditional name) and HMDB accession number or Subclass or UniProt accession number** | **Times Included** | **Number of Iterations** | **Percentage (%)** |
| L-Glutamic Acid | (2R)-2-aminopentanedioic acid (HMDB03339) | 29 | 100 | 29 |
| C18:2 | Unsaturated fatty acids [FA0103] | 80 | 100 | 80 |
| PC(34:3) | Diacylglycerophosphocholines [GP0101] | 35 | 100 | 35 |
| APOE-4 | Apolipoprotein-E (P02649) isoform E4 | 100 | 100 | 100 |

## Claims

1. A diagnostic method to distinguish Alzheimer's patient subjects, comprising determining in a plasma sample of said subject the levels of at least one metabolic marker selected from the list consisting of amino Acids and Biogenic amines Aminoadipic acid (alpha-AAA) and/or Butane-1,4-diamine (putrescine) and comparing the levels of said marker or markers with respect to the levels of the same marker or markers in a HC patient or with respect to a reference value, wherein the subject is classified as suffering AD or as having an increased risk of suffering from AD if the expression levels of Aminoadipic acid are decreased with respect to the levels of the same marker or markers in a HC patient or with respect to a reference value and/or if the levels of putrescine are increased with respect to the levels of the same marker or markers in a HC patient or with respect to a reference value.

2. The diagnostic method of claim 1, wherein the metabolic marker is Aminoadipic acid (alpha-AAA).

3. The diagnostic method of claim 1, wherein the metabolic marker is putrescine.

4. The diagnostic method of claim 1, wherein the method comprises determining the levels of each of the metabolites of the list consisting of aminoadipic acid (alpha-AAA), butane-1,4-diamine (putrescine), arginine, ornithine, glutamic acid, Lyso PC a C18:0, PC aa C34:3, PC ae C30:0, and PC ae C36:0 and the presence of allele APOE4, and comparing the levels of said markers with respect to the levels of the same markers in a HC patient or with respect to a reference value, wherein the subject is classified as suffering AD or as having an increased risk of suffering from AD if there is an increased level(s) of Ornithine, Putrescine, PC aa C34:3, and PC ae C30:0 and the presence of allele APOE4 and a decreased level(s) of L-Arginine, L-Glutamic Acid, Alpha-AAA, Lyso PC a C18:0 and PC ae C36:0.

5. *In vitro* use of a diagnostic kit or device comprising reagents capable of measuring the levels of at least one metabolic marker selected from the list consisting of amino Acids and Biogenic Aminoadipic acid (alpha-AAA) and/or Butane-1,4-diamine (putrescine) in a plasma sample, for distinguish Alzheimer's patient subjects from subjects not suffering from Alzheimer's disease.

6. The use according to claim 5, wherein said kit or device comprises reagents capable of measuring the levels of Aminoadipic acid (alpha-AAA) in a plasma sample.

7. The use according to claim 5, wherein said kit or device comprises reagents capable of measuring the levels of putrescine in a plasma sample.

8. The use according to claim 5, wherein said kit or device comprises reagents capable of measuring the levels of aminoadipic acid (alpha-AAA), butane-1,4-diamine (putrescine), arginine, ornithine, glutamic acid, Lyso PC a C18:0, PC aa C34:3, PC ae C30:0, PC ae C36:0 and the presence of the allele APOE4 in a plasma sample.

9. A method for determining the efficacy of a therapy for AD in a subject, comprising determining in a plasma sample of said subject the levels of at least one metabolic marker selected from the list consisting of amino Acids and Biogenic amines: aminoadipic acid (alpha-AAA) and/or butane-1,4-diamine (putrescine) and comparing the levels of said marker or markers with respect to the levels of the same marker or markers in a HC patient or with respect to a reference value, wherein the therapy is efficient if the expression levels of aminoadipic acid are increased with respect to the levels of the same marker or markers in a HC patient or with respect to a reference value and/or if the levels of putrescine are decreased with respect to the levels of the same marker or markers in a HC patient or with respect to a reference value.

10. The method of claim 9 , wherein the method comprises determining the levels of each of the metabolites of the list consisting of aminoadipic acid (alpha-AAA), butane-1,4-diamine (putrescine), arginine, ornithine, glutamic acid, Lyso PC a C18:0, PC aa C34:3, PC ae C30:0, PC ae C36:0 and the presence of allele ApoE4, and comparing the levels of said markers with respect to the levels of the same markers in a HC patient or with respect to a reference value, wherein the therapy is efficient if there is a decreased level(s) of ornithine, putrescine, PC aa C34:3, PC ae C30:0 and no detection of the presence of allele APOE4 and an increased level(s) of L-Arginine, L-Glutamic Acid, Alpha-AAA, Lyso PC a C18:0 and PC ae C36:0.

11. A method for monitoring the progression of AD in a subject, comprising determining in a plasma sample of said subject the levels of at least one metabolic marker selected from the list consisting of amino Acids and Biogenic amines: aminoadipic acid (alpha-AAA) and/or butane-1,4-diamine (putrescine) and comparing the levels of said marker or markers with respect to the levels of the same marker or markers the same subject at an earlier time point or with respect to a reference value, wherein the disease is progressing if the expression levels of aminoadipic acid are decreased with respect to the levels of the same marker or markers in the same subject at an earlier time point or with respect to a reference value and/or if the levels of putrescine are increased with respect to the levels of the same marker or markers in the same subject at an earlier time point or with respect to a reference value.

12. The method of claim 11 , wherein the method comprises determining the levels of each of the metabolites of the list consisting of aminoadipic acid (alpha-AAA), butane-1,4-diamine (putrescine), arginine, ornithine, glutamic acid, Lyso PC a C18:0, PC aa C34:3, PC ae C30:0, and PC ae C36:0 and the presence of allele APOE4, and comparing the levels of said markers with respect to the levels of the same markers in the same subject at an earlier time point or with respect to a reference value, wherein the disease is progressing if there is an increased level(s) of ornithine, putrescine, PC aa C34:3, PC ae C30:0 and the presence of allele APOE4 and a decreased level(s) of L-Arginine, L-Glutamic Acid, Alpha-AAA, Lyso PC a C18:0 and PC ae C36:0 with respect to the levels of the same marker or markers in the same subject at an earlier time point or with respect to a reference value.

13. *In vitro* use of a kit or device comprises reagents capable of measuring the levels of aminoadipic acid (alpha-AAA), butane-1,4-diamine (putrescine), arginine, ornithine, glutamic acid, Lyso PC a C18:0, PC aa C34:3, PC ae C30:0, PC ae C36:0 and the presence of allele APOE4 in a plasma sample, for the method for determining the efficacy of a therapy for AD in a subject as defined in claim 10.

14. *In vitro* use of a kit or device comprises reagents capable of measuring the levels of aminoadipic acid (alpha-AAA), butane-1,4-diamine (putrescine), arginine, ornithine, glutamic acid, Lyso PC a C18:0, PC aa C34:3, PC ae C30:0, PC ae C36:0 and the presence of allele APOE4 in a plasma simple, for the method for monitoring the progression of AD in a subject as defined in claim 12.

15. A kit or device comprises reagents capable of measuring the levels of aminoadipic acid (alpha-AAA), butane-1,4-diamine (putrescine), arginine, ornithine, glutamic acid, Lyso PC a C18:0, PC aa C34:3, PC ae C30:0, PC ae C36:0 and the presence of allele APOE4 in a plasma sample.
